# EUROPEAN PATENT APPLICATION

(11) **EP 1 398 043 A2**
(43) Date of publication of application: **17.03.2004**
(21) Application number: 03020630.4
(22) Date of filing: 10.09.2003
(51) Int. Cl.: A61L 2/28, A61L 2/16, C09D 5/14

(54) **Process for controlling microbial contamination of polymeric emulsions using carbon dioxide detection**

(30) Priority: 13.09.2002 US 243152
(71) Applicant: AIR PRODUCTS POLYMERS, L.P., Allentown, PA 18195-1501 (US)
(72) Inventor: Rabasco, John, Joseph, Allentown PA 18104 (US); Bott, Richard, Henry, Macungie PA 18062 (US); Schweighardt, Frank, Kenneth, Allentown PA 18104-9558 (US); Sagl, Dennis, Fogelsville PA 18051 (US)
(74) Representative: Kador & Partner

(57) **Abstract**

This invention relates to an improved process for the control of biocontamination in aqueous based polymeric emulsions contained in vessels having a headspace. The improvement comprises monitoring the carbon dioxide concentration in the headspace of the vessel using a direct reading carbon dioxide probe; and, then adding biocide when a preselected concentration of carbon dioxide above the atmospheric carbon dioxide concentration is reached.

## Description

### BACKGROUND OF THE INVENTION

Aqueous based polymer emulsions are comprised of fine organic polymer particles suspended and stabilized in an aqueous environment with either surfactants or protective colloids or a combination of both. Because of the inherent supply of carbon nutrition for microorganisms in these polymer emulsions, they are susceptible to microbial attack and propagation. Such microbial attack and propagation can result in excessive biocontamination and spoilage. A standard industrial practice to combat such product biodeterioration is through the addition of various industrial biocides (antimicrobial agents). Biocides added at the point of manufacture keep the polymeric emulsions free of microbial contaminants for a limited time. But, during storage, microbial contamination can occur and contaminated products having a substantial presence of microbial growth, e.g. above 1 x 10² colony forming units per milliliter, may be unfit for use because of pH changes, viscosity changes, off color, odor and the like and must be scrapped.

The following discussion and references disclose various methods of detecting microbial growth in organic products including polymeric emulsions.

A typical method for determining the presence of microorganisms and quantification of the colony forming units per milliliter (cfu/ml) in polymeric emulsion products is streak plate testing. In streak plating the emulsion polymer is coated onto various agar growth mediums and incubated for 2 to 7 days. The presence of microorganisms can then be determined from the growth on the plate. Absence of growth is an indication that the polymeric emulsion is free of contaminating microorganisms. Because of the time required for microbial detection, excessive biocontamination and biodeterioration can occur during the incubation period, thus wasting valuable time for intervention and product recovery. Further, detection of contamination in polymer emulsions contained in drums or large storage tanks via streak plate testing is dependent upon the location in the container from which the emulsion sample is removed. For example, many microorganisms contaminating polymer emulsions prefer to grow at the surface or upper portions of the emulsion. If the emulsion is sampled from the top of the container, contamination would be detected via streak plate testing. However, if the emulsion is sampled from the bottom of the container, it is likely that streak plate testing would show no contamination. In this example, the presence of contamination would be missed and the emulsion would continue to be biodegraded resulting in lost product.

WO 99/59431 teaches a method for detecting the presence of contaminating bacteria in a food sample stored in cans and other packages by modifying said packages to comprise a hydrophilic polymeric composition lining containing an indicator for the presence of absence of gases, including carbon dioxide. The indicator is triggered by a pH difference reflected by the level of carbon dioxide.

WO 92/12413 teaches the use of a multi-layer body fluid culture sensor comprising a fluorophore embedded in a chemically inert matrix that is transparent at the wavelengths of interest. The fluorophore comprises a pH sensitive absorbance based dye that changes color when the pH is lowered by the reaction of microbial evolved carbon dioxide with water. This method and sensor are used to detect microorganisms in a blood culture bottle.

WO 93/15402 teaches the monitoring of biological activity within a container or bag containing foodstuff or a human thrombocyte concentrate by means of an apparatus for indicating the partial pressure of carbon dioxide. A pH sensitive indicator material is employed to visually indicate elevated carbon dioxide levels.

Given the above state of the art, a need remains for direct analysis of a leading indicator of biological action in the early detection of microbial contaminants in polymeric emulsions. Such early warning of product contamination and the warning of an excessive concentration and growth rate of microorganisms permit immediate intervention to eliminate and prevent subsequent product biodeterioration before the product has lost all commercial value. A further need remains for detecting and responding to the presence of microbial contaminants in polymeric emulsions, on-line, in real time and without removing a sample for off-line analysis. Lastly, a need remains for preventing the contamination of the polymeric emulsions without excessive addition of biocide to the polymeric emulsion.

### BRIEF SUMMARY OF THE INVENTION

This invention relates to an improved process for the identification and control of excess biocontamination in aqueous based polymeric emulsions contained in vessels having a headspace, typically a vessel having a headspace vented to the atmosphere. The improvement for early detection which avoids excessive addition of biocide to the polymeric emulsion comprises: monitoring carbon dioxide concentration in the headspace of the vessel using a direct reading carbon dioxide probe (sensor) ; and, then adding biocide when a preselected concentration of carbon dioxide, above the atmospheric carbon dioxide concentration, is reached. Typically, biocide is added when the carbon dioxide concentration is 100 parts per million (ppm) above atmospheric carbon dioxide concentration (usually 400 ppm v/v).

Several advantages can be achieved by the process described herein and these include:
an ability to detect early on the presence of microorganism growth in the polymeric emulsion and allow for remediation by early application of a suitable biocide;
an ability to estimate the level of microbial activity by the concentration of the carbon dioxide in the headspace;
an ability to avoid the addition of excessive amounts of biocide to the polymeric emulsion;
an ability to detect microorganism activity in real time and from remote locations;
an ability to assess the microbiological quality of polymer emulsions without removing samples from storage vessels; and,
an ability to minimize errors in sampling due to localized growth of microorganisms in the polymer emulsion.

### DETAILED DESCRIPTION OF THE INVENTION

This invention is directed to an improved process for controlling microbial contamination of aqueous based polymeric emulsions in vessels, particularly vessels such as storage tanks, using carbon dioxide sensors that can operate in the humid gas phase matrix and in real time. Direct reading carbon dioxide sensors are used to measure the carbon dioxide concentration in the headspace of the vessel over a period of time. Based upon the carbon dioxide concentration in the headspace of the large storage tank, microbial contamination can be detected. Utilizing the carbon dioxide concentration level and the rate of change of the carbon dioxide concentration over time, the microbial concentration and microbial growth rate may be approximated. Biocide is added in an amount reflective of the approximated level of contamination and growth rate. Early detection and approximation of microbial concentration allows for appropriate response before severe product biodeterioration occurs without adding too much of the biocide. Adding too much biocide may also contribute to the creation of product which is out of specification. Response may be made manually or through automatic means.

Microbial contamination of polymer emulsions can lead to a range of effects, including color changes, odors, viscosity changes, pH changes, and visible surface growth. Excessive contamination, e.g. above 1 x 10² colony forming units per milliliter (cfu/ml) can lead to product spoilage. Examples of microorganisms capable of contaminating polymer emulsions include, but are not limited to, *Aeromonas hydrophilia, Alcaligenes faecalis, Corynebacterium ammoniagenes, Enterobacter aerogenes, Escherichia coli, Klebsiella pneumoniae, Pseudomonas aeruginosa, Proteus vulgaris, Providencia rettgeri, Pseudomonas stutzeri, Shewanella putrefaciens, Serratia liquefaciens, Acinetobacter baumannii, Burkholderia cepacia, Chryseobacterium meningosepticum, Sphingobacterium spiritivorum, Ralstonia pickettii, Gluconoacetobacter liquefaciens, Geotrichum candidum, Aspergillus* species, *Sporothrix* species, *Trichoderma viride, Cladosporium* species, *Rhodoturula glutinis, Candida guillermondi, Penicillium* species, and *Candida tropicalis.*

It has been found that measurement of the concentration and rate of change of concentration of carbon dioxide as a function of volume of polymeric emulsion emitted as a metabolic respiration product provides a unique and unexpected direct measure of microbial contamination and growth rate in the polymeric emulsion. The test mechanism, unlike many techniques, is particularly sensitive to microbial growth in large storage tanks, e.g., 10,000 to 30,000 gallons.

Atmospheric carbon dioxide levels are approximately 300-400 ppm. Low levels of microbial contamination of polymeric emulsions, it is believed, initially is introduced and possibly saturates, the aqueous phase of the polymeric emulsion with carbon dioxide. Then, as the concentration of carbon dioxide in the aqueous phase increases, the carbon dioxide in the headspace begins to increase above atmospheric levels. Carbon dioxide concentrations exceeding background levels by a specified amount, e.g., 100 ppm, has allowed for the establishment of criteria by which contamination can be identified and approximated. For example, bacterial contamination levels as low as 1 x 10² cfu/ml have been observed in experiments to result in a doubling of headspace carbon dioxide concentrations to approximately 600-800 ppm. Thus, once carbon dioxide is detected at a level 100 ppm above the concentration in normal air, the concentration, and the rate of change of carbon dioxide concentration are used to alert operators or automatic dispensers to intervene and provide treatment before spoilage occurs.

The carbon dioxide sensors that can be used in this invention must withstand the temperature, humidity, pH and other process conditions likely to be found in emulsion polymer storage containers. Using these sensors, polymeric emulsions retained in storage tanks, rail cars, tank trucks, and other areas of possible contamination can be monitored for the presence of microorganisms through the detection of the carbon dioxide as a respiration product and for the rate of change, in real time. Preferred carbon dioxide sensors are carbon dioxide transmitters incorporating CARBOCAP® sensors. The CARBOCAP sensor is a single-beam dual-wavelength NDIR sensor. These sensors are well suited to withstand harsh and humid environments. These sensors are capable of measuring a wide range of carbon dioxide concentrations, up to 20%. They can provide data in minutes, and on an ongoing, real. time basis, which can allow for less labor, more complete data ensuring the integrity of the products, and the ability to intervene and stop the contamination prior to biodeterioration of the polymeric emulsion. The carbon dioxide concentration can be monitored at a location remote from the vessel and sensor, using a transmission mechanism, such as wireless technology, telephone lines, or the internet.

Once microbial activity is detected, excessive biodeterioration of the polymeric emulsions can be prevented by the addition of various biocides (antimicrobial agents). Examples of commonly used industrial biocides are: hydrogen peroxide, 1,2-benzisothiazolin-3-one (BIT), and a blend of 5-chloro-2-methyl-4-isothiazolin-3-one (CIT) and 2-methyl-4-isothiazolin-3-one (MIT). Examples of other biocides commonly used for polymer emulsion preservation include 1,2-dibromo-2,4-dicyanobutane (DBDCB), 2,2-dibromo-3-nitrilo-propionamide (DBNPA), 2-bromo-2-nitro-1,3-propanediol (BNPD), aldehyde derivatives, formaldehyde releasing agents, hydantoins, and chlorinated aromatics. Examples of cationic biocides that are particularly effective in preserving polymer emulsions that have been stabilized with protective colloids, such as poly(vinyl alcohol), against biodeteriogenic microbes are: substituted pyridinium salts, substituted guanidine salts, tetrasubstituted ammonium salts, and polymeric cationic compounds, in which the substitution can be an alkyl, a cycloalkyl, and/or an aryl group of 2 to 18 carbons. The cationic compounds are also particularly effective in preserving polymer emulsions with low VOC's (i.e. less than 1000 ppm VOC).

Polymeric emulsions susceptible to microbial attack and treatable per the procedures described herein include essentially all dispersions of synthetic polymers and copolymers in aqueous media. Examples of polymeric emulsions formed by the emulsion polymerization of monomers which include vinyl acetate, ethylene and other olefins, diolefins such as butadiene, various alkyl acrylates, various alkyl methacrylates, styrene, vinyl chloride, vinyl esters, acrylamides, methacrylamides, N-methylolacrylamides, maleates, and others known in the art. Examples of polymer emulsions for purposes of this invention, then, include emulsions of poly(vinyl acetate), poly(vinyl acetate) copolymers such as poly(vinyl acetate-co-ethylene) (VAE), poly(vinyl acetate-acrylics) such as poly(vinyl acetate-butyl acrylate) and poly(vinyl acetate-(2-ethyl)hexyl acrylate), polyacrylics, polymethacrylics, poly(styrene-acrylics), wherein acrylics can include C₃₋₁₀ alkenoic acids, such as acrylic acid, methacrylic acid, crotonic acid and isocrotonic acid and their esters, other polystyrene copolymers, poly(vinyl chloride-co-ethylene) copolymers, and the like.

Surfactants and protective colloids employed in the formation of the polymeric emulsions include anionic, cationic and nonionic surfactants such as ethoxylated alkyl phenols, dialkyl esters of sulfonic acids, block ethylene/propylene oxide copolymers and so forth. Protective colloids commonly used include hydroxyethyl cellulose and poly(vinyl alcohol).

Polymeric emulsions may also be in a formulated state. By that, it is meant that the aqueous polymeric emulsion can be combined with pigments as in paint formulations. The can be formulated with fillers and tackifiers as in adhesive formulations. By the term "polymeric emulsion" then, it is meant to include the polymeric emulsion obtained by emulsion polymerization and its formulated state.

The following examples are intended to be illustrative of various embodiments of the invention.

### EXAMPLE 1

### CONTROL

Sterile AIRFLEX® 400 (1600 grams) poly(vinyl alcohol) stabilized vinyl acetate/ethylene polymeric emulsion was placed in a 2L plastic Nalgene container. The plastic cover was modified with two ports. The first port was used to mount and insert the carbon dioxide sensor and the second, much smaller diameter port was left open to the atmosphere to permit venting and a constant pressure within the container. A Vaisala GMT222 direct reading carbon dioxide transmitter connected to a data logger and computer was used as the measuring device. The cover was then screwed on tightly and the headspace carbon dioxide concentration measured over the course of 3 days. During this entire monitoring period the headspace carbon dioxide concentration remained steady at approximately 400 ppm.

When the polymeric emulsion is streak tested, no microbial growth is observed in the control.

### EXAMPLE 2

### CARBON DIOXIDE MEASUREMENT OF CONTAMINATED EMULSION

In an effort to determine the efficacy of carbon dioxide measurement as a detection mechanism for microbial growth, AIRFLEX 400 (1600 grams) poly(vinyl alcohol) stabilized (some biocide present) vinyl acetate/ethylene polymeric emulsion was inoculated with *Gluconoacetobacter liquefaciens.* The resulting concentration of *Gluconoacetobacter liquefaciens* in the Airflex 400 emulsion was measured via a dilution plate count on potato dextrose agar and found to be 5.4 x 10⁴cfu/ml. The contaminated emulsion was placed in a 2L plastic Nalgene container. The modified cover, equipped with the carbon dioxide sensor and vent hole, described in Example 1 was tightly screwed on. The headspace carbon dioxide level began rising above background (-400 ppm) within a matter of minutes. After continually monitoring this sample for 2 days, the headspace carbon dioxide concentration leveled out at about 1600 ppm.

### EXAMPLE 3

### CARBON DIOXIDE MEASUREMENTS AFTER BIOCIDE ADDITION

To the same contaminated sample described in Example 2 was added 400 ppm of dodecylguanidine hydrochloride (DGH) biocide to reduce the Gluconoacetobacter liquefaciens concentration.

The sample was then monitored for headspace carbon dioxide concentration as described in Examples 1 and 2. After continually monitoring this sample for 2 days the headspace carbon dioxide concentration leveled out at about 1150 ppm. A sample of the AIRFLEX 400 poly(vinyl alcohol) stabilized vinyl acetate/ethylene polymeric emulsion was removed from the container and the concentration of *Gluconoacetobacter liquefaciens* was measured on potato dextrose agar using a dilution plate count method. The AIRFLEX 400 emulsion was found to have a *Gluconoacetobacter liquefaciens* contamination level of 1.30 x 10⁴ cfu/ml.

This Example shows that the reduction of carbon dioxide in the headspace indicated that the biocide addition was effective in reducing the microbial growth from that of Example 2. The results also show that as the rate of carbon dioxide reduction began to taper off (the rate of reduction slows) at the 1150 ppm, thus illustrating that microbial growth remained and that additional biocide was required. One need not wait until equilibrium occurs to determine that a substantial level of microbial growth remained. A slowing of the rate of carbon dioxide reduction at a level of carbon dioxide above about 300 ppm atmospheric levels, even 1000 ppm, is indicative of a need for additional biocide.

The results might likely be more dramatic if a quick kill biocide (such as hydrogen peroxide or bleach) had been used. DGH is not a quick kill biocide. This example, however, is useful to show that the treatment is having an effect solely on the basis of the measured carbon dioxide concentration in the headspace of the vessel. Thus, in a commercial setting, once elevated CO2 is detected, intervention can occur to fix the problem before it gets out of hand.

### EXAMPLE 4

### BIOCIDE ADDITION AND CARBON DIOXIDE MEASUREMENT

To the same contaminated sample described in Example 3 was added an additional 375 ppm DGH to further reduce the *Gluconoacetobacter liquefaciens* concentration. The sample was then monitored for headspace carbon dioxide concentration as described in Examples 1 and 2. After continually monitoring this sample for 3 days the headspace carbon dioxide concentration leveled out at about 1000 ppm. A sample of the AIRFLEX 400 poly(vinyl alcohol) stabilized vinyl acetate/ethylene polymeric emulsion was removed from the container and the concentration of *Gluconoacetobacter liquefaciens* was measured on potato dextrose agar using a dilution plate count method. The AIRFLEX 400 emulsion was found to have a *Gluconoacetobacter liquefaciens* contamination level of 4.40 x 10³ cfu/ml.

As in Example 3, the carbon dioxide concentration was instructive in alerting one that microbial growth remained, although in lower concentration than in Example 3, but still too high for a commercial product.

### EXAMPLE 5

### BIOCIDE ADDITION AND CARBON DIOXIDE MEASUREMENT

To the same contaminated sample described in Example 4 was added an additional 350 ppm DGH to further reduce the *Gluconoacetobacter liquefaciens* concentration. The sample was then monitored for headspace carbon dioxide concentration as described in Examples 1 and 2. After continually monitoring this sample for 3 days the headspace carbon dioxide concentration leveled out at about 800 ppm. A sample of the AIRFLEX 400 emulsion was removed from the container and the concentration of Gluconoacetobacter liquefaciens was measured on potato dextrose agar using a dilution plate count method. The AIRFLEX 400 poly(vinyl alcohol) stabilized vinyl acetate/ethylene polymeric emulsion was found to have a *Gluconoacetobacter* liquefaciens contamination level of 3.20 x 10² cfu/ml.

The results of Examples 1-5 can be illustrated in the following table and graph:

| Example | CO₂, ppm | Contamination, CFU/ml | Biocide, ppm | Time, hour |
|---|---|---|---|---|
| 1 | 0 | 0 | 0 | 0 |
| 2 | 1600 | 5.4 X 10⁴ | 0 | 24 |
| 2 inoculation | 1600 | 5.4 X 10⁴ | 400 | Start |
| 3 | 1150 | 1.3 X 10⁴ | 400 | 24 |
| 4 | 1000 | 4.4 X 10³ | 375 | 96 |
| 5 | 800 | 3.2 X 10² | 350 | 96 |

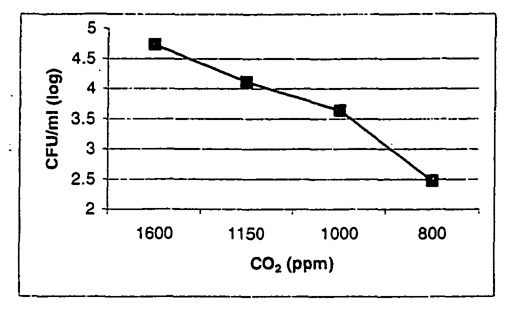

In summary, Examples 1-5 show that the concentration of carbon dioxide in the headspace of a vented storage vessel can be used to detect microbial growth in the emulsion. The concentration of carbon dioxide in the headspace at conditions of near equilibrium can be an indication of microbial growth and suggest appropriate levels of biocide necessary to reduce the microbial growth. The declining rate of carbon dioxide concentration in the headspace affords an opportunity to predict whether further addition of biocide is necessary. A leveling of the carbon dioxide concentration above atmospheric is an indication that more biocide is required.

## Claims

1. In a process for the control of biocontamination in aqueous based polymeric emulsions contained in vessels having a headspace, the improvement for early detection and avoiding excessive addition of biocide to the aqueous based polymeric emulsion which comprises:
direct monitoring of carbon dioxide concentration in the headspace of the vessel using a direct reading carbon dioxide sensor; and, then adding a biocide when a preselected concentration of carbon dioxide above the atmospheric carbon dioxide concentration is reached.

2. The process of Claim 1 wherein the biocide is added when the carbon dioxide concentration in the headspace of the vessel is 100 parts per million (ppm) above atmospheric carbon dioxide concentration.

3. The process of Claim 2 wherein biocide is added when the rate of carbon dioxide reduction slows down at 300 ppm above atmospheric carbon dioxide concentration.

4. The process of Claim 1 wherein the headspace of the vessel is vented to the atmosphere.

5. The process of claim 1 wherein the carbon dioxide concentration is monitored at a location remote from the vessel and sensor.

6. The process of claim 1 wherein the carbon dioxide concentration is monitored at a location remote from the vessel and sensor, using wireless technology, telephone lines, or the internet.
